Europäisches Patentamt

European Patent Office

Office européen des brevets

(11) Publication number: **0 278 246 B1**

(12) **EUROPEAN PATENT SPECIFICATION**

(45) Date of publication of patent specification: **12.06.91**  (51) Int. Cl.⁵: **B01J 2/12**, C07C 37/00, C07C 37/84

(21) Application number: **88100412.1**

(22) Date of filing: **14.01.88**

(54) Production of granular bisphenols.

(30) Priority: **20.01.87 US 5504**

(43) Date of publication of application:
**17.08.88 Bulletin 88/33**

(45) Publication of the grant of the patent:
**12.06.91 Bulletin 91/24**

(84) Designated Contracting States:
**BE CH DE ES FR GB IT LI NL SE**

(56) References cited:
**DE-B- 1 102 173**
**FR-A- 1 485 300**
**FR-A- 2 440 774**
**US-A- 3 694 170**

(73) Proprietor: **THE DOW CHEMICAL COMPANY**
**2030 Dow Center Abbott Road P.O. Box 1967**
**Midland Michigan 48640-1967(US)**

(72) Inventor: **McDonald, Kenneth T.**
**202 Larkspur**
**Lake Jackson Texas 77566(US)**
Inventor: **Shirley, Arthur R., Jr.**
**202 Shirley**
**Florence Alabama 35633(US)**

(74) Representative: **Sternagel, Hans-Günther, Dr.**
**et al**
**Patentanwälte Dr. Michael Hann Dr. H.-G.**
**Sternagel Sander Aue 30**
**W-5060 Bergisch Gladbach 2(DE)**

## Description

This invention relates to a process for granulating bisphenols.

Bisphenols are currently available in the form of prills, flakes and crystals. Bisphenols in the form of prills, flakes or crystals have a tendency to break up and form dust. Thus, during production of the various forms, the product is difficult to convey and has the potential to form high levels of dust which can be explosive.

Prills of bisphenols are generally produced by solidifying small droplets of bisphenol product in a relatively large diameter and tall tower. The prills produced by these processes are generally porous and inherently weak and thus, are easily crushed in solids handling systems. The prill process using towers is inefficient and requires a high volume of air to solidify the bisphenol. Large filtration units are generally required to clean the incoming air. Foreign particles that pass through the filtration system can contaminate the product. Bisphenol dust and vapor has to be removed from this large volume of air as it exits the system. Relatively expensive equipment is needed in the prill process. A disadvantage of the prill process is that there is a significant product yield loss. In addition, oxygen in contact with hot bisphenols is known to increase the color of the product. Another disadvantage of this process is its potential for a dust explosion.

Flakes of bisphenol are generally produced by solidifying bisphenols on a cool surface and then crushing the resultant solids. A large amount of dust is formed by the crusher during the flake process. In addition, the flat particles (flakes) do not flow very well and have a high angle of repose. The characteristics of the flakes inherently create material handling problems.

Crystallized products of bisphenols are inherently weak and friable, thus a lot of dust is created during handling of these particles. In addition, the crystal product does not flow out of hoppers very easily.

It is desired to provide a process for granulating bisphenols which eliminates many of the process deficiencies of the prill, flake and crystal processes. It is further desired to produce bisphenol granules that have and retain excellent handling characteristics such as high crush strength and low friability which permits the product to be easily handled, for example, unloading from bulk containers and hopper cars, without forming dangerously explosive levels of dust.

The present invention is a process for producing bisphenol granules by feeding a seed material of bisphenol into a rotary granulation drum enclosed in a gastight housing and spraying molten bisphenols onto the seed material in the presence of an inert gaseous atmosphere.

## Brief Description of the Drawings

Figure 1 is a schematic flow diagram showing the granulation process of the present invention.

Figure 2 is a cross-section of the granulation drum in Figure 1 taken along the line 2-2.

"Granulation" herein means the gradual increase of undersized nuclei to product size by successive layering or coating of the nuclei with the same or dissimilar molten compounds.

The bisphenol product of the present invention is produced by a melt granulation process wherein granules of the required size are grown by spraying molten bisphenol directly onto seed material. Bisphenol normally is a solid or semisolid at ambient temperatures but can be reduced to a sprayable liquid state by melting and can be returned to the solid or semisolid state by cooling below the melt temperature. Generally, the granules of bisphenols have a high crush strength since each particle develops from a single seed and each new layer is melted into the outer layer of the bead it coats thus producing a nonporous bead that has a glazed outer layer which resists dusting.

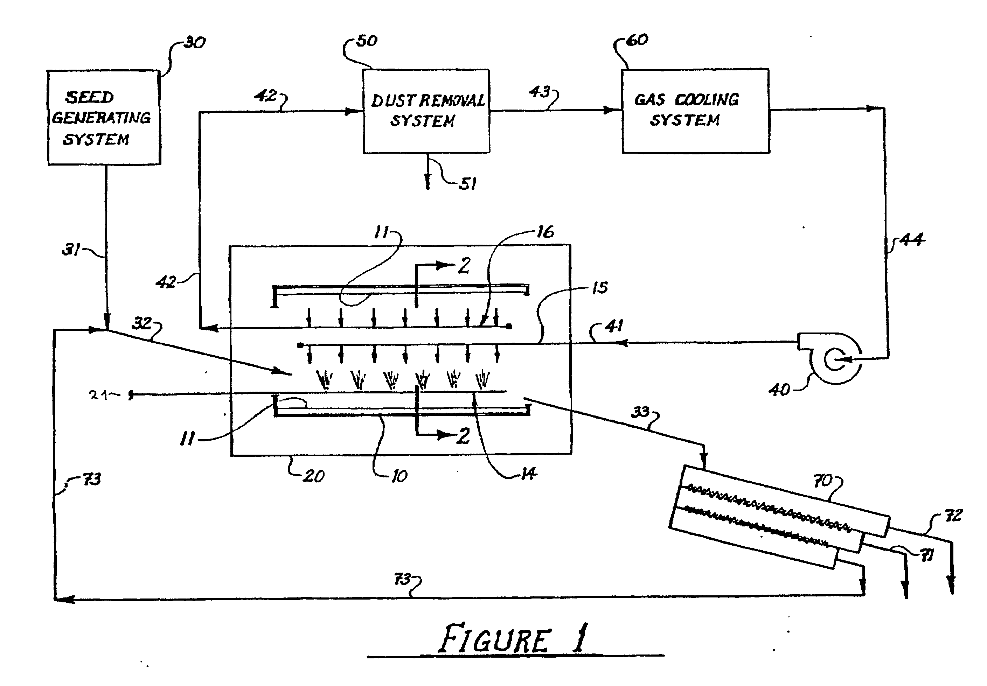

In Figure 1, there is shown one embodiment of the process of the present invention for melt granulation of bisphenols fed into a rotary granulation drum 10 through feed stream 21. Granulation occurs in the rotary drum 10 enclosed in a gastight enclosure 20 for operating the drum in a gaseous inert atmosphere. The enclosed unit can be advantageously run at a slight vacuum to avoid dust blowing out of the enclosure 20 and into the atmosphere and polluting the environment.

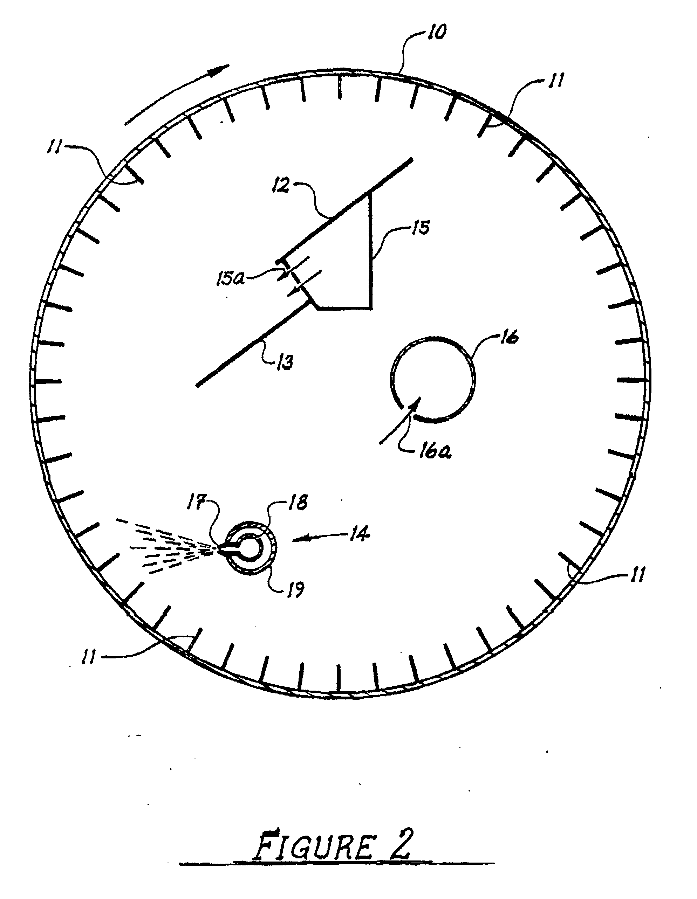

The rotary drum 10 used in the present invention is of the type described in U.S. Patent Nos. 3,877,4125; 3,991,225; 4,213,924; 4,424,176 and 4,506,453. The drum 10 of the present invention is more clearly shown in Figure 2.

With reference to Figure 2, the rotary drum of the present invention contains specially designed internal equipment including lifting vanes or flights 11, collecting or deflector pans 12 and 13, spray header and nozzle assembly 14, gas distributors 15 and gas collection header pipes 16, as herein described. Generally, in the rotary drum 10 the longitudinal lifting vanes, or flights 11, preferably are equally spaced on the drum's inside shell and specially designed to form continuous longitudinal curtains of falling solid particles. The shower of falling solid particles formed by the movement of the lifting flights 11 fall on the deflector pan or pans 12 and 13 for providing a curtain of falling granules on which the molten material can be sprayed with the spray header and nozzle assembly 14.

Seed particles of bisphenols produced by any conventional seed generating system 30 are fed into the drum granulator 10. The seed particles can be produced, for example, using conventional crushing or milling equipment. Preferably, recycled undersize granules produced in the present process, shown in Figure 1 as stream 73 are mixed with the seed particles, shown as stream 31, and then the mixture in stream 32 is fed into the granulation drum 10 as seed material to form a bed of material in the drum. The mixing of the undersize granules and the seed particles can be carried out in a conventional feed hopper with a metering means for controlling the feed such as a weigh feeder (not shown).

As the drum 10 rotates, the seed particles and recycle undersize granules are elevated from the bed by the lifting flights 11 and dropped onto the two inclined collecting pans 12 and 13 installed in a step fashion. The rotational speed of the drum is preferably from about 5 rpm (revolutions per minute) to about 18 rpm. Granules flowing from the top pan 12 onto the bottom pan 13 form an upper curtain and granules flowing from the bottom pan 13 into the flights 11 form a dense bottom curtain. Molten bisphenol is preferably sprayed on the lower curtain of the falling granules.

The preferred operating temperature of the undersized beads in the granulator is from 70° to 138°C and more preferably, from 123°C to 130°C. At temperatures lower than 70°C, an undesirable quantity of small beads, i.e., beads ranging from 200 to 400 micron size are produced. As the temperature of the beads increases, the production of small beads decreases and some of the seed particles agglomerate. The surface of these beads may be dull. At above 138°C a tacky dust is formed and within a few minutes, the dust forms undesirable large balls ranging from 2.54 to 3.81 cm in diameter.

The bisphenol hot melt feed rate of the process is dependent on the desired capacity, for example, a capacity of 2268 kg (5000 pounds) per hour of melt feed can be used. The hot melt temperature is preferably from 156°C to 180°C and more preferably from 158°C to 165°C. The hot melt temperature is maintained at from 2°C to 10°C above melting of the bisphenol.

Spraying of molten bisphenols onto the falling granules is carried out using a plurality of spray nozzles 17 and tube members 18 and 19 supported in the header assembly 14. Any spray header system with a plurality of spray nozzles can be used in the present process to spray molten bisphenols onto the seed material fed into the drum granulator 10. For example, a pipe or tube with nozzles attached along its length can be used for feeding liquid bisphenols to the drum and onto the seed material.

Preferably, a steam jacketed spray header and nozzle assembly 14 is used to maintain the temperature of the melt. The spray pressure used is from 446 to 1480 kPa (50 psig to 200 psig). The spray pattern is linear with each nozzle rotated slightly off the horizontal to avoid over lapping spray streams. Preferably, the spray nozzles each have inlet screens for minimizing nozzle plugging.

The droplets of molten bisphenols should be atomized finely enough that the formation of agglomerates in the granulation drum is minimized. The formation of finely atomized spray is preferably carried out by use of pressure-atomizing nozzles, i.e., nozzles in which the liquid is propelled by its own pressure through a small orifice at such high velocity that the stream is broken down into small droplets. The finely atomized bisphenols melt forms a thin layer on the falling granules, melts into the solid surface and quickly solidifies on the relatively cool surface. Product granules of the desired size are formed by successive layering of the melt on the granules as they pass through the drum 10.

As the sprayed bisphenols solidifies on the undersize granules, considerable heat is released into the granulation drum. Cooling is provided by an inert gas stream being directed through the upper curtain of falling granules through gas distributors 15 passing through outlets 15a, thus providing an efficient method of heat exchange between the hot granules and the cooled gas stream. Efficient utilization of the cooling gas makes it practical to enclose the system with enclosure 20 and recirculate the inert gas.

The inert gaseous stream, in this instance nitrogen, is passed from a blower 40 through stream 41 to the gas distributor 15 inside the granulator drum 10. The gas exit header 16 with inlets 16a is used to pull the nitrogen from the drum to a dust removal means 50 through stream 42. Dust from the granulator 10 is separated from the nitrogen leaving the granulator in stream 42, for example, in a cyclone separator or bag house filters. The dust collected in stream 51 from the dust removal means 50 may be remelted and the melt may be recycled to the melt bisphenol feed stream 21 or the melt may be sent to a use point.

Preferably, the nitrogen stream 43 from the dust removal system is cooled using a gas cooling means 60. For example, the cooling means may be a conventional shell and tube-type heat exchanger using cooling tower water as the cooling medium. Preferably the nitrogen stream is cooled down to a temperature of from 25°C to 60°C. Then, the nitrogen in stream 44 can be recycled back to the granulator 10 after passing through the blower 40.

The nitrogen flow rate is dependent upon the desired capacity, for example for a unit with a capacity of 2268 kg (5000 pounds) per hour requires a flow rate of from 2.83 to 3.78 m³/s is small when compared to a prill system which is typically from 9.44 to 11.8 m³/s (20,000 SCFM to 25,000 SCFM) for a system with an equivalent capacity. The totally enclosed system of the present invention substantially minimizes yield loss, substantially reduces color of the product and substantially removes the potential for dust explosion.

In the process of the present invention relatively little dust is formed. It is believed that the dust levels generated in the present system range from 10 mg/m³ to 50 mg/m³. Any dust produced in the granulation drum which exits the drum with the gaseous stream can be separated from the gas collected by conventional equipment known in the art, as aforementioned. In addition to the above collection process, it is preferred to remove as much dust from the gaseous stream within the granulation drum prior to the gaseous stream exiting the drum through the exhaust header 15.

Granules in stream 33 discharge from the drum granulator 10 into a double-deck screen 70 for separating the product from oversize and undersize granules. The onsize or product granules of bisphenols (shown as stream 71 in Figure 1) are routed from the screen 70 to, for example, bulk storage, to a fluid bed cooler for further cooling, or to a desired use point. The oversize (stream 72) from the screen 70 may be remelted in a melter (not shown) and recycled or fed into the granulator 10 with the molten bisphenol feed stream 21, or conveyed to a desired use point. Optionally, the oversize may be ground for use as seed material. The undersize (stream 73) from the screen 70 is conveyed to a recycle hopper (not shown), where it is fed back into the granulation drum with seed material at a metered rate by a weigh feeder. Optionally, the undersize may be fed into the melter for recycling or conveyed to another desired use point.

The granules of bisphenol produced by the method of the above described process have excellent physical characteristics for providing beads which are capable of being handled more readily and easily without creating dangerous dust levels. The beads are substantially rounded but not necessarily perfect spheres. Any particles size above about 1 millimeter (mm) can be produced by granulation. Normally, the particle size of the onsize granules is from 1 mm to 4 mm, preferably 1.5 mm to 2.5 mm in diameter.

The crush strength of the beads is measured by the force per unit area. Crush strength of beads is determined by the size and quality of the beads. The quality is determined by several factors among which are the strength of the material, shape, and voids in the beads. Beads of uniform quality would be expected to break when the stress, force per unit area, reaches a yield value. In this situation the crush strength would be expected to be proportional to the square of the bead diameter of bead mass raised to the 2/3 power. The crush strength of the bisphenols granules is generally from 300 to 500 grams per square millimeter. Generally, the crush strength numbers are obtained by first measuring the diameter of a bead and then placing the bead on an analytical balance such as a Metler balance. A force is then applied on the bead until the bead yields. The following equation can be used to obtain the crush strength:

$$\text{Crush Strength} = \frac{\text{Force}}{(D^2)2/3}$$

The friablility of the beads is measured by the percent (%) breakage of beads rotating in a drum containing 3/8 inch (0.95 cm) steel balls. The friablility of the bisphenols granules is generally from about 6 percent to about 10 percent. When the granules are allowed to cool slowly, the granules have better friability than those that are rapidly cooled in a fluid bed. The friability test shows that the former has about a 3% breakage whereas the latter has about a 9% breakage. Thus, it is only necessary to rapidly cool the product that is to be placed into sacks immediately.

The bulk density of the bisphenols product is generally from 625 to 657 kg/m³ (39 pounds per cubic foot (lb/ft³) to 41 lb/ft³).

Another advantage of the present process is that it reduces the moisture content of the bisphenols. The moisture content of the bisphenols is generally from 1000 ppm to 300 ppm.

In this granulation process a rotary drum is loaded with previously made seed or recycle material. The temperature of this system is brought up to 120° C. As the drum rotates, the solid material is lifted by flights and discharged on the deflecting pans of the drum. As the material falls off the top pan, the resultant curtain of beads is contacted by 45° C nitrogen being recirculated through the drum at the rate of 750 SCFM (0.35 m³/s). The cooled material is joined by other material falling off the flights onto the lower deflecting pan which gathers granules together and discharges them in a falling curtain in front of the liquid spray header. The granules are sprayed with 160° C Bisphenol A which is forced through the spray nozzle at a pressure of 790 kPa (100 psig) and at a rate of 453 kg (1000 pounds) per hour. The hot liquid bisphenol coats the granules with a thin layer which solidifies as it melts into the previous coating. The liquid solidifies before being contacted by other recently sprayed granules, thus preventing agglomerations. The granules overflow out of the drum and are conveyed to screens where the product size material is separated from the undersized material. The undersized material is recycled at a rate of 906 kg (2000 pounds) per hour to the granulator where it is joined with new seed material which is added at a rate of 22.68 kg (50 pounds) per hour. The process is then repeated.

## Claims

1. A process for granulating bisphenols comprising:

    (a) feeding a seed material of bisphenols to a rotary granulation drum, said drum being sealed to the atmosphere in a gastight manner;

    (b) spraying molten bisphenols onto the seed material falling in a curtain-like pattern inside the drum; and

    (c) substantially simultaneously with steps (a) and (b) feeding an inert gaseous stream into the drum.

2. The process of Claim 1 including the step of screening the product discharging from the rotary drum.

3. The process of Claim 2 including the step of removing dust generated in the rotary drum from the inert gaseous stream discharging from the drum.

4. The process of Claim 1 including the step of recycling the inert gaseous stream discharging from the drum back into the drum.

5. The process of Claim 4 including the step of cooling the inert gaseous stream prior to feeding the stream into the drum.

6. The process of Claim 1 wherein the melt feed temperature is at from 156°C to 180°C.

7. The process of Claim 1 wherein the pressure of the enclosed drum is maintained at slightly below atmospheric pressure.

8. The process of Claim 1 wherein the inert gaseous stream is nitrogen.

9. The process of Claim 5 wherein the gaseous stream is cooled to a temperature of from 25°C to 60°C.

10. The process of Claim 1 wherein the granulation product from the drum ranges from 1 mm to 4 mm in size.

## Revendications

1. Procédé de granulation de bisphénols, consistant à :

    (a) introduire des germes de bisphénols dans un tambour de granulation rotatif, ledit tambour étant isolé, de façon étanche aux gaz, de l'atmosphère environnante;

    (b) pulvériser des bisphénols fondus sur les germes tombant en rideau à l'intérieur du tambour; et

    (c) amener dans le tambour, pratiquement simultanément aux étapes (a) et (b), un courant de gaz inerte.

2. Procédé selon la revendication 1, comprenant l'étape consistant à cribler le produit déchargé du tambour rotatif.

3. Procédé selon la revendication 2, comprenant l'étape consistant à séparer la poussière formée dans le tambour rotatif d'avec le courant de gaz inerte sortant du tambour.

4. Procédé selon la revendication 1, comprenant l'étape consistant à recycler dans le tambour le courant de gaz inerte sortant du tambour.

5. Procédé selon la revendication 4, comprenant l'étape consistant à refroidir le courant de gaz inerte avant l'amenée du courant dans le tambour.

6. Procédé selon la revendication 1, dans lequel la température d'alimentation de la substance fondue est de 156°C à 180°C.

7. Procédé selon la revendication 1, dans lequel la pression du tambour enfermé est maintenue légèrement au-dessous de la pression atmosphérique.

8. Procédé selon la revendication 1, dans lequel le courant de gaz inerte est constitué d'azote.

9. Procédé selon la revendication 5, dans lequel le courant gazeux est refroidi à une température de 25°C à 60°C.

10. Procédé selon la revendication 1, dans lequel le produit de granulation sortant du tambour présente une taille de 1 mm à 4 mm.

## Ansprüche

1. Verfahren zum Granulieren von Bisphenolen durch

   (a) Einbringen eines Keimmaterials von Bisphenolen in eine drehende Granuliertrommel, wobei die Trommel gegenüber der Atmosphäre gasdicht verschlossen ist,

   (b) Aufsprühen von geschmolzenen Bisphenolen auf das im Inneren der Trommel in einem vorhangähnlichen Muster herabfallende Keimmaterial und

   (c) Einbringen eines Inertgasstromes in die Trommel, im wesentlichen gleichzeitig mit den Schritten (a) und (b).

2. Verfahren nach Anspruch 1,
   **dadurch gekennzeichnet,**
   daß das aus der drehenden Trommel abgezogene Material gesiebt wird.

3. Verfahren nach Anspruch 2,
   **dadurch gekennzeichnet,**
   daß der aus der Trommel abgezogene Inertgasstrom von dem in der drehenden Trommel entwickelten Staub befreit wird.

4. Verfahren nach Anspruch 1,
   **dadurch gekennzeichnet,**
   daß der aus der Trommel abgezogene Inertgasstrom in die Trommel zurückgeführt wird.

5. Verfahren nach Anspruch 4,
   **dadurch gekennzeichnet,**
   daß der Inertgasstrom vor dem Einbringen in die Trommel gekühlt wird.

6. Verfahren nach Anspruch 1,
   **dadurch gekennzeichnet,**
   daß das in geschmolzenem Zustand eingebrachte Material eine Temperatur von 156 °C bis 180 °C aufweist.

7. Verfahren nach Anspruch 1,
   **dadurch gekennzeichnet,**
   daß der Druck in der abgeschlossenen Trommel geringfügig unter Atmosphärendruck gehalten wird.

8. Verfahren nach Anspruch 1,
   **dadurch gekennzeichnet,**
   daß der Inertgasstrom Stickstoff ist.

9. Verfahren nach Anspruch 5,
   **dadurch gekennzeichnet,**
   daß der gasförmige Strom auf eine Temperatur von 25 °C bis 60 °C gekühlt wird.

10. Verfahren nach Anspruch 1,
    **dadurch gekennzeichnet,**
    daß das aus der Trommel erhaltene Granulat eine Größe von 1 mm bis 4 mm aufweist.

SEED GENERATING SYSTEM

DUST REMOVAL SYSTEM

GAS COOLING SYSTEM

FIGURE 1

FIGURE 2